# EUROPEAN PATENT APPLICATION

(11) **EP 1 813 585 A1**
(43) Date of publication of application: **01.08.2007**
(21) Application number: 05806319.9
(22) Date of filing: 14.11.2005
(51) Int. Cl.: C07B 63/04, C07C 7/20, C07C 15/44, C07C 15/46

(54) **METHOD OF INHIBITING POLYMERIZATION GIVING COPOLYMER OF DIVINYLBENZENE AND AROMATIC VINYL COMPOUND**

(30) Priority: 15.11.2004 JP 2004330363
(71) Applicant: Hakuto Co., Ltd, Shinjuku-ku, Tokyo 160-8910 (JP)
(72) Inventor: TANIZAKI, Seiji, c/o Yokkaichi Laboratory, Yokkaichi-shi, Mie, 510-0007 (JP); NAKAJIMA, Junichi, c/o Yokkaichi Laboratory, Yokkaichi-shi, Mie 510-0007 (JP)
(74) Representative: Kramer - Barske - Schmidtchen
(86) International application number: PCT/JP2005/020817
(87) International publication number: WO 2006/051941

(57) **Abstract**

[PROBLEMS] To provide: a method by which the generation of a copolymer of an aromatic vinyl compound and either the divinylbenzene generated in the production of the aromatic vinyl compound or the divinylbenzene which has come into the system together with a raw material for the aromatic vinyl compound is inhibited in the steps of distilling, purifying, and storing the aromatic vinyl compound: and a method of inhibiting the copolymer from depositing on and thus fouling devices and facilities.

[MEANS FOR SOLVING PROBLEMS] The method of inhibiting the generation of a copolymer of divinylbenzene and an aromatic vinyl compound is **characterized in that** a dinitrophenol compound (A), a sulfonic acid compound (B), and a p-phenylenediamine compound (C) are simultaneously used in the steps of producing, purifying and storing the aromatic vinyl compound.

## Description

### TECHNICAL FIELD

The present invention relates to a polymerization inhibiting method of inhibiting the formation of a copolymer of an aromatic vinyl compound and divinylbenzene which has been generated in the production of the aromatic vinyl compound, in the steps of producing and purifying the aromatic vinyl compound.

### BACKGROUND ART

Styrene and derivatives thereof as aromatic vinyl compounds are industrially very important chemical compounds as raw materials for polystyrene, synthetic rubber, ABS resin, etc., and have been mass-produced industrially.

Generally, aromatic vinyl compounds have been obtained by carrying out dehydrogenation of corresponding alkylbenzene in the presence of catalysts. For example, in the case of styrene, ethylbenzene has been brought into contact with inorganic catalysts to obtain styrene, and obtained styrene has been separated from ethylbenzene with distillation, and purified. At this time, a small amount of divinylbenzene is also generated along with styrene. Divinylbenzene has a boiling point higher than that of styrene so that divinylbenzene has been separated from styrene in the steps of distilling and purifying styrene, mostly remained in a distillation still, and condensed therein. Divinylbenzene has two vinyl groups in each molecular and acts as a cross-linking agent. Therefore, copolymers in which divinylbenzene and styrene are cross-linked to each other have been formed during the production of styrene, and deposited in interiors of devices as fouling to cause a serious problem in the operations of devices and facilities for use in the production of styrene. These cross-linked polymers exhibit low solubility against organic solvents so that when once deposited in the interiors of the devices, they must be removed by hand. Therefore, the removing works have been carried out periodically, and the improvement thereof has been strongly desired.

Under the above circumstances, in order to restrain the formation of the copolymer in which divinylbenzene and styrene are cross-linked to each other, prevent the depositing of formed fouling of the copolymer in devices and facilities for use in the production of styrene, and accordingly prevent the occurrence of a serious problem in the operations of the devices for use in the production of styrene, polymerization inhibitors against styrene and derivatives thereof along with polymerization inhibiting methods using these polymerization inhibitors have been applied. More specifically, there are polymerization inhibitors and polymerization inhibiting methods against styrene, which use phenol compounds, nitrosophenol compounds, and nitrophenol compounds (see Patent document 1, ex.), methods which use piperidine-1-oxyl compounds (see Patent document 2, ex.) methods which use nitrophenol compounds and piperidine-N-oxyl compounds (see Patent document 3, ex.), etc. However, in these polymerization inhibiting methods, a large amount of polymerization inhibitor has been used, but satisfactory effects on the polymerization inhibition against copolymers in which divinylbenzene and aromatic vinyl compounds are cross-linked to each other cannot be exhibited, and accordingly, the improvement thereof has been strongly desired.

Patent document 1: Publication of unexamined Japanese patent application No. Sho63-316745
Patent document 2: Publication of unexamined Japanese patent application No. Hei1-165534
Patent document 3 Publication of unexamined Japanese patent application No. Hei6-166636

### DISCLOSURE OF THE INVENTION

### PROBLEM TO BE SOLVED WITH THE INVENTION

The present invention has an object of providing a method of inhibiting the formation of a copolymer of an aromatic vinyl compound and divinylbenzene which has been generated in the production of the aromatic vinyl compound or has come into the system together with a raw material for the aromatic vinyl compound in the steps of distilling, purifying and storing the aromatic vinyl compound, and a method of inhibiting the copolymer from depositing on and thus fouling devices, facilities, etc.

### MEANS FOR SOLVING PROBLEM

The present inventors have been thoroughly investigated on the polymerization reaction of copolymers containing divinylbenzene and aromatic vinyl compounds, and found that by simultaneously using a dinitrophenol compound, a sulfonic acid compound and a p-phenylenediamine compound in combination, formation of copolymers in which divinylbenzene and aromatic vinyl compounds are cross-linked to each other is remarkably inhibited, and have completed the present invention.

Nemely, the invention claimed in claim 1 is a polymerization inhibiting method giving a copolymer of divinylbenzene and an aromatic vinyl compound,
characterized in that, in the steps of producing, purifying and storing the aromatic vinyl compound, a dinitrophenol compound (A), a sulfonic acid compound (B) and a p-phenylenediamine compound (C) expressed by the formula (1) wherein R¹, R², R³ and R⁴ are respectively a hydrogen atom, a methyl group, an ethyl group and an alkyl group which may form straight chains, branched chains or three to eight membered rings composed of three to twenty carbon atoms, are used simultaneously.

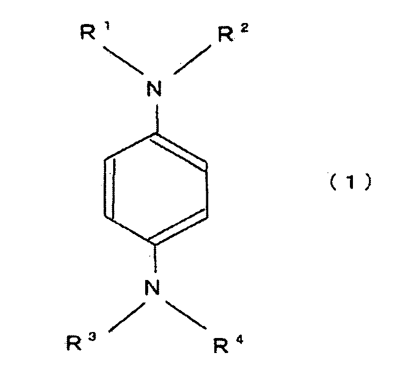

The invention claimed in claim 2 is the polymerization inhibiting method giving the copolymer of divinylbenzene and the aromatic vinyl compound, which is claimed in claim 1, and is characterized in that the dinitrophenol compound (A) is composed of at least one compound selected from the group consisting of 2, 4 dinitrophenol, 2, 6- dinitrophenol, 2, 6-dinitro-4-methylphenol, 2, 4-dinitro-6-methylphenol and 2, 4-dinitro-6-sec-butylphenol.

The invention claimed in claim 3 is the polymerization inhibiting method giving the copolymer of divinylbenzene and the aromatic vinyl compound, which is claimed in claim 1 or 2, and is characterized in that the sulfonic acid compound (B) is composed of at least one compound selected from the group consisting of toluene sulfonic acid, xylene sulfonic acid, cumene sulfonic acid, dodecylbenzene sulfonic acid, pentadecylbenzene sulfonic acid and dinonylnaphthalene sulfonic acid.

The invention claimed in claim 4 is the polymerization inhibiting method giving the copolymer of divinylbenzene and the aromatic vinyl compound, which is claimed in one of claims 1 through 3, and is characterized in that the p-phenylenediamine compound (C) is composed of at least one compound selected from the group consisting of N-phenyl-N-isopropyl-p-phenylenediamine, N, N-di-sec-butyl-p-phenylenediamine, N, N-di-phenyl-p-phenylenediamine, N, N-di-2-naphthyl-p-phenylenediamine and N-phenyl-N-(1,3-dimethylbutyl)-p-phenylenediamine.

The invention claimed in claim 5 is the polymerization inhibiting method giving the copolymer of divinylbenzene and the aromatic vinyl compound, which is claimed in one of claims 1 through 4, and is characterized in that the component (A), the component (B) and the component (C) are added such that the composition weight ratio of the component (B) to the component (C) ranges from 90 : 10 to 40 : 60, and the weight ratio of the component (A) to the total amount of the component (B) and the component (C) ranges from 99 : 1 to 50 : 50.

### EFFECTS OF THE INVENTION

In accordance with the method of the present invention, there are exhibited such economical effects that in the steps of producing, purifying and storing an aromatic vinyl compound, formation of a cross-linked copolymer of the aromatic vinyl compound and divinylbenzene which has been generated or come into the system in the steps of producing and purifying the aromatic vinyl compound is inhibited, the generation and the depositing of fouling in devices and facilities, which are caused by the copolymer, are inhibited, the reduction and the improvement of conventional works of removing the copolymer are effected, the maintenance of the process flow and the thermal conduction along with continuous operations over a long period of time become possible, and the improvement of the safe operations, products yield, and products quality can be effected.

### BEST MODE FOR CARRYING OUT THE INVENTION

The present invention relates to a method of inhibiting the formation of a cross-linked copolymer of an aromatic vinyl compound and divinylbenzene which has been generated in the step of producing the aromatic vinyl compound, in the steps of producing, purifying and storing the aromatic vinyl compound, and a method of inhibiting fouling caused by the depositing of the above-described copolymer on devices and facilities in the steps of producing, purifying and storing the aromatic vinyl compound.

In the present invention, examples of the aromatic vinyl compound include styrene and styrene derivatives having polymeric vinyl groups, such as alkylstyrene having alkyl groups with one to ten carbon atoms, α-alkylstyrene having phenyl groups and alkyl groups with one to ten carbon atoms at α positions, β-alkylstyrene having phenyl groups and alkyl groups with one to ten carbon atoms at βpositions, etc. More specifically, examples thereof include styrene, methylstyrene (ortho-isomer, meta-isomer, para-isomer and a mixture thereof), ethylstyrene (ortho-isomer, meta-isomer, para-isomer and a mixture thereof), propylstyrene (ortho-isomer, meta-isomer, para-isomer and a mixture thereof), butylstyrene (ortho-isomer, meta-isomer, para-isomer and a mixture thereof), octylstyrene (ortho-isomer, meta-isomer, para-isomer and a mixture thereof), nonylstyrene (ortho-isomer, meta-isomer, para-isomer and a mixture thereof), decylstyrene (ortho-isomer, meta-isomer, para-isomer and a mixture thereof), α-methylstyrene (2-phenylpropene) (cis-isomer, trans-isomer and a mixture thereof), 1-phenylpropene (cis-isomer, trans-isomer and a mixture thereof), 2-pnenyl-2-butene (cis-isomer, trans-isomer and a mixture thereof), stilbene (cis-isomer, trans-isomer and a mixture thereof), etc.

The objective steps in the present invention include the step of producing the aromatic vinyl compound, the step of purifying the aromatic vinyl compound after produced, and the step of storing and holding the produced and purified aromatic vinyl compound in which divinylbenzene is contained, along with attachment devices, equipments, etc. adapted to contact process liquids containing divinylbenzene and the aromatic vinyl compound in respective steps, and a circulatory system and a recycle system including the above-described devices and equipments. More specifically, such steps include dehydrogenation columns and synthesis columns for an alkyl aromatic compound in the step of producing the aromatic vinyl compound, reactants outlet lines and recovery and recycle lines after reaction, feed lines of the aromatic vinyl compound to purification columns, preheating lines, cooling lines and recycling lines in the purifying step, holding tanks and storage tanks in the storing step, transfer tanks, transport tanks, transfer lines thereof, etc.

Examples of divinylbenzene in accordance with the present invention include divinylbenzene which has been generated as a by-product when the aromatic vinyl compound is produced, and divinylbenzene which has come into the system together with a raw material adapted to be used upon producing the aromatic vinyl compound, and divinylbenzene may take any configuration such as meta-configurations, para-configurations or a mixture thereof.

The copolymer of divinylbenzene and the aromatic vinyl compound in accordance with the present invention (hereinafter, will be referred to as "copolymer") is the copolymer which contains divinylbenzene having two polymeric vinyl groups along with aromatic vinyl compounds, and has a cross-linked structure. Examples thereof include a divinylbenzene-styrene copolymer. The copolymer having a cross-linked structure is deposited on devices and lines, which are located in the steps from the producing step through the storing step of the aromatic vinyl compound, and grows to form fouling.

The dinitrophenol compound used in the present invention is the phenol compound having two nitro groups in each molecule, and examples thereof include 2,4-dinitrophenol, 2,4-dinitro-6-methylphenol, 2,4-dinitro-6-ethylphenol, 2,4-dinitro-6-propylphenol, 2,4-dinitro-6-isopropylphenol, 2,4-dinitro-6-n-butylphenol, 2,4-dinitro-6-sec-butylphenol, 2,4-dinitro-6-tert-butylphenol, 2,6-dinitrophenol, 2,6-dinitro-4-methylphenol, 2,6-dinitro-4-ethylphenol, 2,6-dinitro-4-propylphenol, 2,6-dinitro-4-isopropylphenol, 2,4-dinitro-6-n-butylphenol, 2,6-dinitro-4-sec-butylphenol, 2,6-dinitro-4-tert-butylphenol, etc., and at least one of these compounds is used.

Examples of the sulfonic acid compound used in the present invention include alkylbenzene sulfonic acid and alkylnaphthalene sulfonic acid in which sulfonic acid groups are combined with benzene rings and naphthalene rings. More specifically, they include toluene sulfonic acid, xylene sulfonic acid, cumene sulfonic acid, dodecylbenzene sulfonic acid, pentadecylbenzene sulfonic acid, dinonylnaphtalene sulfonic acid, etc., and at least one of these compounds is used.

The p-phenylenediamine compound used in the present invention is the p-phenylenediamine compound expressed by the formula (1). In this formula, R¹, R², R³ and R⁴ are respectively a hydrogen atom, a methyl group, an ethyl group, and an alkyl group which may form straight chains, branched chains or three to eight membered rings composed of three to twenty carbon atoms. More specifically, N-phenyl-N-isopropyl-p-phenylenediamine, N,N-di-sec-butyl-p-phenylenediamine, N,N-di-phenyl-p-phenylenediamine, N,N-di-2-naphthyl-p-phenylenediamine, N-phenyl-N-(1,3-dimethylbutyl)-p-phenylenediamine, etc. are examples thereof, and at least one of these compounds can be used.

The weight ratio of the dosage of the sulfonic acid compound (B) and that of the p-phenylenediamine compound (C) in the polymerization inhibiting method of the present invention ranges from 90 : 10 to 40 : 60, preferably ranges from 80 : 20 to 50 : 50, and more preferably ranges from 70 : 30 to 60 : 40. When the ratio of the dosage of the sulfonic acid compound (B) to that of the p-phenylenediamine compound (C) is outside this range, there may be the cases effects of the present invention cannot be sufficiently exhibited. In addition, the weight ratio of the dosage of the dinitrophenol compound (A) to the total dosage of the sulfonic acid compound (B) and the p-phenylenediamine compound (C) ranges from 99 : 1 to 50 : 50, preferably ranges from 95 : 5 to 75 : 25, and more preferably ranges from 90 : 10 to 80 : 20. When the ratio of the dosage of the dinitrophenol compound (A) to the total dosage of the sulfonic acid compound (B) and the p-phenylenediamine compound (C) is outside this range, there may be the cases effects of the present invention cannot be sufficiently exhibited.

The total dosage of the dinitrophenol compound (A), the sulfonic acid compound (B) and the p-phenylenediamine compound (C) depends on the conditions of objective steps, necessary inhibition degree of formation of polymerization, etc. and is not determined equally, but in general, normally ranges from 10 to 10,000 ppm, preferably ranges from 50 to 5,000 ppm, and more preferably ranges from 100 to 3,000 ppm relative to objective styrene. This dosage has been found as a suitable range for the exhibition of the objective polymerization inhibiting effects against the copolymer, and when the dosage is less than this range, such effects may not be sufficiently exhibited, and when the dosage is greater than this range, effects are sufficiently exhibited, but such improvement of the polymerization inhibiting effects as to correspond to the dosage may not be obtained, which is less preferable from the economical point of view.

The adding method of the dinitrophenol compound, the sulfonic acid compound and the p-phenylenediamine compound in accordance with the present invention is not limited specifically, but, normally, the method of adding them to a specific position together, the method of adding them to several positions, or other methods are arbitrarily selected. In any case, there is the method of adding the dinitrophenol compound, the sulfonic acid compound and the p-phenylenediamine compound separately, or the method of adding the dinitrophenol compound, the sulfonic acid compound and the p-phenylenediamine compound after dissolving them in the same liquid as the process fluid, such as ethylbenzene and crude styrene in case of styrene, ex. in a proper mixing ratio. Any one of these methods will do.

In the present invention, other well known polymerization inhibitors may be used together provided that the effects of the present invention are not damaged therewith, and the present invention does not limit the use of these well known polymerization inhibitors.

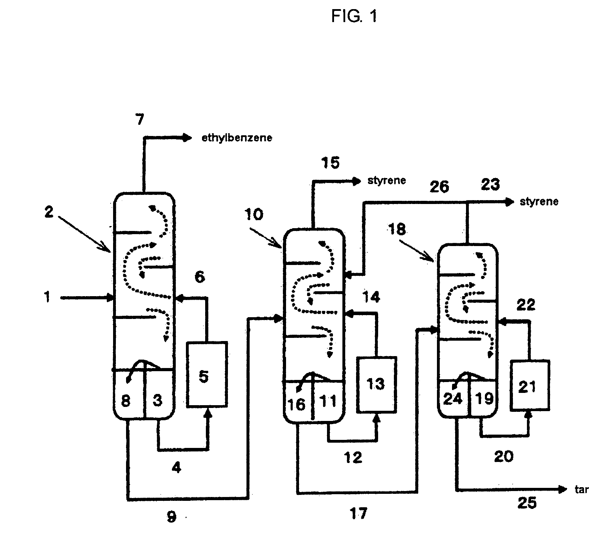

Hereinafter, the polymerization inhibiting method giving the copolymer in accordance with the present invention will be explained with reference to the production of styrene by the dehydrogenation of ethylbenzene (see FIG. 1).

Styrene is formed by the dehydrogenation of ethylbenzene using catalysts, and a mixture of the formed styrene and ethylbenzene is fed to a central part of a distillation column (2) as a feed oil (1). In the distillation column (2), unreacted ethylbenzene contained in the supplied feed oil (1) is separated and recovered from a column top (7) of the distillation column, and is used in the dehydrogenation again. On the other hand, both styrene of which the boiling point is higher that that of ethylbenzene and a small amount of divinylbenzene gather in a column bottom of the distillation column as a bottom liquid. The column bottom of the distillation column is normally divided to two sections, that is a bottom liquid gathering section (3) and a recovering and gathering section (8) for a bottom liquid which has overflowed from the bottom liquid gathering section (3) and acts as a feed oil to a next distillation column (10). This bottom liquid gathering section (3) is connected to a reboiler (5) located adjacent to the distillation column (2) via a line (4), and the reboiler (5) is connected to the central part of the distillation column (1) via a line (6). The bottom liquid gathered in the bottom liquid gathering section (3) flows in the reboiler (5) via the line (4) provided in the column bottom thereof, is heated therein to be evaporated, and enters the distillation column (1) again. Consequently, ethylbenzene of which the boiling point is as low as 136 °C is distilled and separated, and remaining substances of which the boiling points are high return to the column bottom of the distillation column, again. Normally, the reboiler is composed of a bundle of many metallic narrow tubes. The bottom liquid flows in the narrow tubes, and the exterior of the narrow tubes is heated with steam, etc. to the temperatures higher than the boiling point (136°C) of ethylbenzene.

The bottom liquid containing styrene and a small amount of divinylbenzene, which has gathered in the recovering and gathering section (8) provided in the column bottom of the distillation column, is guided to the central part of the distillation column (10) via a line (9) as a feed oil for use in the next process, and styrene (boiling point: 145 °C) contained therein is distilled and separated at a column top (15) of the distillation column. Undistilled components contained therein flow down and are gathered in a gathering section (11) located at the bottom of the distillation column. Then, they flow to a reboiler (13) via a line (12), are heated therein to the temperatures higher than the boiling point of styrene to be evaporated, and enter the distillation column again from the central part of the distillation column (10) via a line (14). Residual styrene is distilled and separated again at a column top (15). And the bottom liquid containing styrene and a small amount of divinylbenzene, which has gathered in a recovering and gathering section (16) located at the column bottom of the distillation column (10), is supplied via a line (17) as a feed oil for use in a second styrene distillation column (18). In the second styrene distillation column (18), styrene is recovered from a column top (23) of the distillation column, similarly to the preceding process, and at the same time, the obtained styrene is returned to the central part of the preceding distillation column (10) via a line (26), and distilled again therein. By connecting a plurality of distillation columns to each other and repeating the distilling operations, both the recovery of ethylbenzene and the recovery as well as the purification of formed styrene are carried out. On the other hand, distillation residue gathered in the recovering and gathering sections located at the column bottoms of the distillation columns are supplied as feed oils for use in the distillation columns in the following processes, but, finally disposed of (in many cases, subjected to combustion treatment) as tar components.

On the other hand, copolymers are formed in the distilling step. It is known that in gas-liquid interfaces within the metallic narrow tubes of the reboiler, of which the temperature becomes highest, copolymers in which styrene and divinylbenzene are cross-linked to each other may be formed mostly in a short time. The formed copolymers cannot be distilled so as to gather at the column bottoms of the distillation columns, and gradually increase during the repetition of the distilling and purifying steps. Consequently, the concentration of copolymers may become as high as about 30 % by weight at the column bottom (including the bottom liquid gathering section and the recovering and gathering section) of the final distillation column in the distilling step.

In accordance with the present invention, the adding positions of the dinitrophenol compound, the sulfonic acid compound and the p-phenylenediamine compound are not limited specifically, but normally, they are added in the step upstream of the positions where divinylbenzene and the aromatic vinyl compound are copolymerized to form a cross-linked substance. For example, in the case of the production of styrene by the above-described dehydrogenation of ethylbenzene, there are the method of adding these compounds to mixtures subjected to the dehydrogenation of ethylbenzene, the method of adding these compounds to feed oils for use in the distillation columns, which are adapted to separate styrene after the dehydrogenation of ethylbenzene, the method of adding these compounds in the distillation columns adapted to distill styrene, the method of adding them in the bottom liquid gathering sections of the distillation columns, the method of injecting them in lines adapted to connect the column bottoms of the distillation columns and the reboilers to each other, etc., and any method will do. Amine compounds to be used in the present invention include compounds of which the boiling points are lower than that of styrene, but by using the method of adding the above-described compounds in the bottom liquid gathering sections of the distillation columns, or the method of injecting them in lines adapted to connect the column bottoms of the distillation columns and the reboilers to each other, the effects thereof can be exhibited without being affected by the lower boiling point than styrene.

### EMBODIMENTS

The present invention will be explained in more detail with reference to embodiments, but the present invention is not limited to the following embodiments.

### (dinitrophenol compounds)

DNBP: 2,4-dinitro-6-sec-butylphenol
DNP: 2,4-dinitrophenol
DNOC: 2,4-dinitro-6-methylphenol
DNPC: 2,6-dinitro-4-methylphenol

### (sulfonic acid compounds)

PTS: para toluene sulfonic acid
XSA: xylene sulfonic acid
CSA: cumene sulfonic acid
DDBSA: dodecylbenzene sulfonic acid,
PDBSA: pentadecylbenzene sulfonic acid
DNNSA: dinonylnaphthalene sulfonic acid.

### (p-phenylenediamine compounds)

PPPA: N-phenyl-N-isopropyl-p-phenylenediamine
BPA: N, N-di-sec-butyl-p-phenylenediamine ["UOP#5"(trade name), manufactured by UOP LLC]
PPA: N, N-di-phenyl-p-phenylenediamine
NPA: N, N-di-2-naphthyl-p-phenylenediamine

### [Polymerization inhibition test]

Styrene monomer, divinylbenzene(a mixture of meta-isomer and para-isomer), ethylvinylbenzene, diethylbenzene were respectively washed with alkali to remove polymerization inhibitors contained in each of these materials, washed with water and dried. 50.0 g of styrene monomer, 27.5 g of divinylbenzene (a mixture of meta-isomer and para-isomer), 11.3 g of ethylvinylbenzene, and 11.3 g of dimethylbenzene were put in a four necked separable flask provided with a reflux condenser, a polymerization inhibitor was added thereto by a predetermined amount relative to the entire amount of materials (the composition ratio of each material was shown in TABLE 1), and a high purity nitrogen gas was supplied therein for 30 minutes to remove residual oxygen, thereby preparing a testing liquid. The testing liquid was introduced in a test tube (weight : Ag) within a double tube which has been heated to 210 °C with a quantitative pump according to the JIS K 2276 Jet Fuel Thermal Oxidation Test (JFTOT method), and the liquid temperature was kept at 180°C. After 90 minutes, the heating of the test tube was finished, the test tube was taken from the double tube, and the weight of the test tube (weight: Bg) on which resin has deposited was measured. The amount of the deposit formed by heating the testing liquid was determined as "B - A" (g). In the present test, in the case of the testing liquid which does not contain divinylbenzene, the resin formed is polystyrene, and polystyrene is dissolved in the testing liquid which has been used in the present test so as not to deposit on the test tube. On the other hand, the divinylbenzene-styrene polymer is the polymer with a cross-linked structure. Therefore, it is not dissolved in the testing liquid so as to deposit on the test tube as deposit. The test results were shown in TABLE 1.

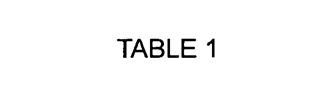

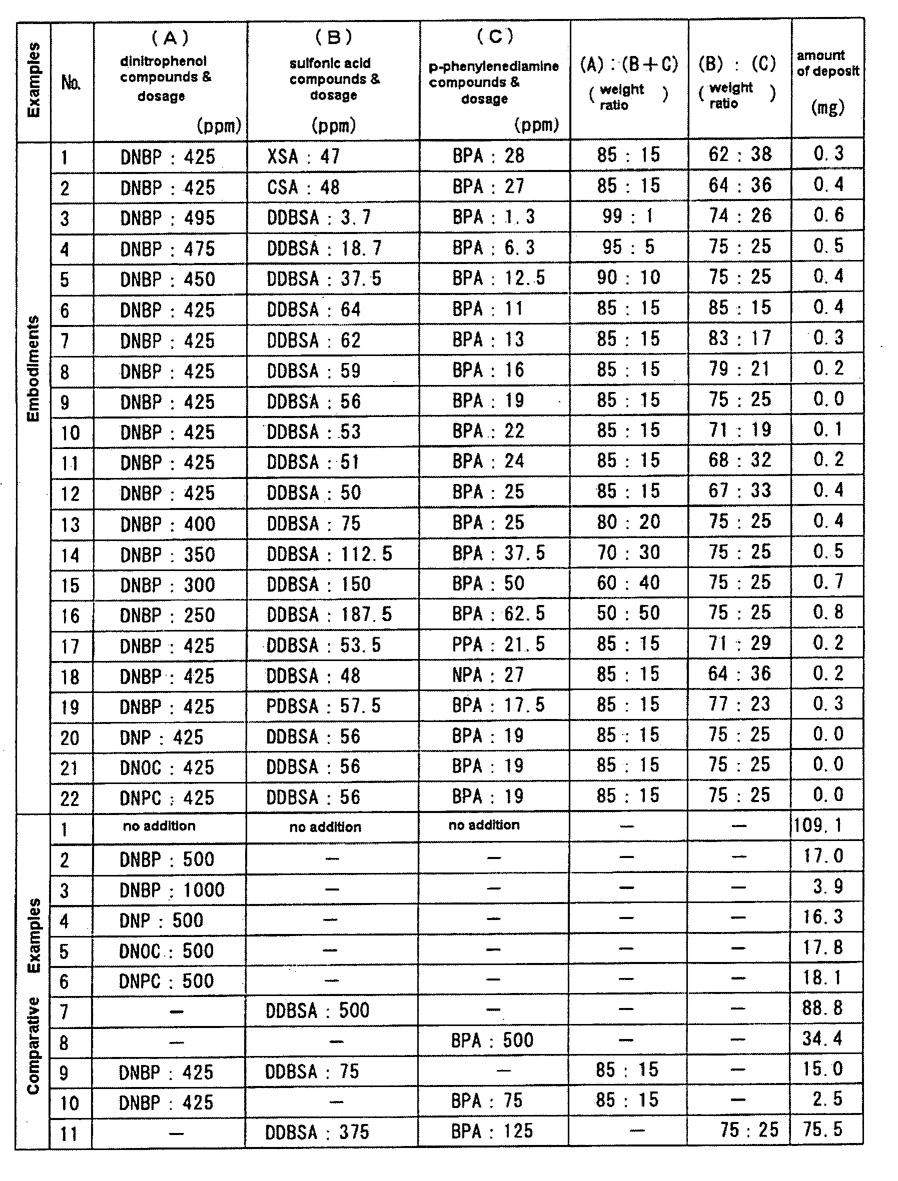

In accordance with the present invention, by combining the dinitrophenol compound, the sulfonic acid compound and the p-phenylenediamine compound with each other, the polymerization inhibition effects against the styrene-divinylbenzene copolymer can be exhibited greatly, as compared with the conventional cases where the dinitrophenol compound is used alone, and the amount of deposit reduces beyond our expectations. These results clearly show that high fouling restraining effects are exhibited.

### BRIEF DESCRIPTION OF DRAWING

FIG. 1 is a separation · purification flow with distillation of ethylbenzene-styrene.

### EXPLANATION OF REFERENCE NUMBER

- 1:: styrene-contained feed oil after dehydrogenation of ethylbenzene
- 2:: distillation column (1)
- 3:: bottom liquid gathering section
- 4:: pipe 1
- 5:: reboiler (1)
- 6:: pipe 2
- 7:: top of distillation column
- 8:: recovering and gathering section of distillation column (1)
- 9:: pipe (3)
- 10:: distillation column (2)
- 11:: gathering section located at the bottom of distillation column (2)
- 12:: pipe (4)
- 13:: reboiler (2)
- 14:: pipe (5)
- 15:: top of distillation column (2)
- 16:: recovering and gathering section of distillation column (2)
- 17:: pipe (5)
- 18:: distillation column (3)
- 19:: bottom liquid gathering section of distillation column (3)
- 20:: pipe (7)
- 21:: reboiler (3)
- 22:: pipe (8)
- 23:: top of distillation column (3)
- 24:: recovering and gathering section of distillation column (3)
- 25:: pipe (8)
- 26:: pipe (9)

## Claims

1. A polymerization inhibiting method giving a copolymer of divinylbenzene and an aromatic vinyl compound, **characterized in that**, in the steps of producing, purificating and storing the aromatic vinyl compound, a dinitrophenol compound (A), a sulfonic acid compound (B) and a p-phenylenediamine compound (C) expressed by the formula (1) wherein R¹, R², R³ and R⁴ are respectively a hydrogen atom, a methyl group, an ethyl group and an alkyl group which may form straight chains, branched chains or three to eight membered rings composed of three to twenty carbon atoms, are used simultaneously.

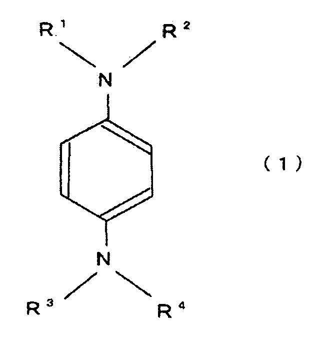

2. The polymerization inhibiting method giving the copolymer of divinylbenzene and the aromatic vinyl compound, which is claimed in claim 1, wherein the dinitrophenol compound (A) is composed of at least one compound selected from the group consisting of 2,4-dinitrophenol, 2,6-dinitrophenol, 2, 6-dinitro-4-methylphenol, 2,4-dinitro-6-methylphenol and 2,4-dinitro-6-sec-butylphenol.

3. The polymerization inhibiting method giving the copolymer of divinylbenzene and the aromatic vinyl compound, which is claimed in one of claims 1 and 2, wherein the sulfonic acid compound (B) is composed of at least one compound selected from the group consisting of toluene sulfonic acid, xylene sulfonic acid, cumene sulfonic acid, dodecylbenzene sulfonic acid, pentadecylbenzene sulfonic acid and dinonylnaphthalene sulfonic acid.

4. The polymerization inhibiting method giving the copolymer of divinylbenzene and the aromatic vinyl compound, which is claimed in one of claims 1 through 3, wherein the p-phenylenediamine compound (C) is composed of at least one compound selected from the group consisting of N-phenyl-N-isopropyl-p-phenylenediamine, N,N-di-sec-butyl-p-phenylenediamine, N,N-di-phenyl-p-phenylenediamine, N,N-di-2-naphthyl-p-phenylenediamine, and N-phenyl-N-(1,3-dimethylbutyl)-p-phenylenediamine.

5. The polymerization inhibiting method giving the copolymer of divinylbenzene and the aromatic vinyl compound, which is claimed in one of claims 1 through 4, wherein the component (A), the component (B) and the component (C) are added such that the composition weight ratio of the component (B) to the component (C) ranges from 90 : 10 to 40 : 60, and the weight ratio of the component (A) to the total amount of the component (B) and the component (C) ranges from 99 : 1 to 50 : 50.
